# EUROPEAN PATENT SPECIFICATION

(11) **EP 1 079 799 B1**
(45) Date of publication and mention of the grant of the patent: **14.07.2004**
(21) Application number: 99926403.9
(22) Date of filing: 21.05.1999
(51) Int. Cl.: A61K 7/48, A61K 7/06

(54) **USE OF AN ANTIMICROBIAL AGENT**
VERWENDUNG EINES ANTIMIKROBIELLEN MITTELS
UTILISATION D'UN AGENT ANTIMICROBIEN

(30) Priority: 21.05.1998 GB 9810799
(43) Date of publication of application: 07.03.2001
(73) Proprietor: The Boots Company PLC, Nottingham, Nottinghamshire NG2 3AA (GB)
(72) Inventor: de GRAAF, Thalie, Paulina, Nottingham NG2 3AA (GB); GALLEY, Edward, Nottingham NG2 3AA (GB); BUTCHER, Kate Elizabeth, Nottingham NG2 3AA (GB)
(74) Representative: Jones, Stephen Anthony
(86) International application number: PCT/EP1999/003592
(87) International publication number: WO 1999/059540

(56) References cited:
- EP-A- 0 603 627
- GB-A- 2 184 356
- GB-A- 2 233 227
- PATENT ABSTRACTS OF JAPAN vol. 015, no. 060 (C-0805), 13 February 1991 (1991-02-13) & JP 02 289506 A (SAKAI CHEM IND CO LTD), 29 November 1990 (1990-11-29)
- TICHY S: "TRANSPARENT ZNO FOR SKIN- AND SUNPROTECTION" SOFW-JOURNAL SEIFEN, OELE, FETTE, WACHSE, vol. 119, no. 8, 8 June 1993 (1993-06-08), pages 487-490, XP000370086 ISSN: 0942-7694 cited in the application
- DATABASE WPI Section Ch, Week 9616 Derwent Publications Ltd., London, GB; Class A26, AN 96-157291 XP002115277 & JP 08 041379 A (KAO CORP), 13 February 1996 (1996-02-13)

## Description

This invention teaches the use of a modified form of zinc oxide as an agent to inhibit microbial growth.

Micro-organisms, present upon the skin exacerbate many minor human afflictions, such as acne, dandruff and athletes foot. Despite the considerable amount of research conducted upon these subjects, these are still common complaints. Efforts have focused upon developing agents which will kill off the micro-organisms, and are suitable for application to the human body. Not only do micro-organisms continuously develop resistance but many successful antimicrobials cause burning or irritation to the skin. Thus, there is an enormous need for new antimicrobials.

This invention discloses the new use of a modified form of zinc oxide as an antimicrobial agent. The zinc oxide has a surface area between 30m²/g and 100m²/g, preferably greater than 90m²/g. The zinc oxide particles used in this invention have a diameter between 0.1 and 200µm. Hereinafter, such zinc oxide particles shall be known as "high surface area zinc oxide". The surface area of the material can be determined using laser diffraction. From the surface area, the mean estimated spherical diameter may be calculated. The diameter of the particles is henceforth taken to mean the mean estimated spherical diameter. The surface area of the high surface area zinc oxide particles used with this invention was calculated using a Micromeritics Flavsorb II 2300 BET apparatus. Such a zinc oxide is commercially available from Elementis as Activox C80.

The production of such zinc oxides is well documented.

### WO 95/04704 (Harcros)

This patent discloses a method for the production of zinc oxide in the form of discrete particles which have an average particle size of 0.08µm or less in diameter and a surface area >12.5m²/g. The zinc oxide produced is found to be particularly good as an additive for scattering/absorbing UV light.

### S. Tichy, SOFW - Journal 119 Jahrgang 8/93

This article teaches a method for producing zinc oxide with a particle size of 20nm and a surface area of 50-150m²/g.

### Liu et al - Journal of Materials Science 21 (1986) 3698-3702

This describes another method of producing zinc oxide particles with a diameter of 0.15µm and a surface area of about 50m²/g.

These articles are but a small selection from a large number of articles detailing the manufacture of high surface area zinc oxide particles.

EP-A-603627 discloses a process for preparing microfine zinc oxide particle diameter is 5 to 100 nm and BET-surface area of from 30 to 100m²/g for use as a sunscreen. There is no disclosure of any antimicrobial properties.

Patent Abstracts of Japan of JP-A-02289506 discloses zinc oxide having surface area of 20-30 m²/g and particle size 0.1 µm (100 nm) as a cosmetic ingredient but there is no disclosure of antimicrobial properties.

GB-A-2184356 discloses the use of microfine zinc oxide of average particle size of from 70-300 nm as a sunscreening agent. There is, however, no disclosure of the surface area nor of antimicrobial activity.

Surprisingly, it has been found that high surface area zinc oxide is a potent agent for stopping microbial growth. *In vitro* trials have demonstrated its great efficacy against the micro-organisms responsible for a number of common human afflictions. However, for them to be effective in use, they must be applied to the body.

Many antimicrobials are harsh reagents that cause burning and irritation to human skin, and so are unsuitable for application to the human body. High surface area zinc oxide is a mild reagent which does not cause burning and irritation to the skin. Thus it is suitable for incorporation into formulations designed to be applied to human skin.

Because of its high potency and because it does not irritate the skin, high surface area zinc oxide may be included in formulations designed to treat some of the more common human afflictions which are microbial in origin or exacerbated by subsequent microbial growth.

Derwent Abstract of 96-157291 of JP-A-8041379 discloses surface treated zinc oxide of surface area 15-100 m²/g which has the "effect of solidifying sebum" but no particle size is given.

Microbes are commonly taken to mean any organism too small to be seen unless by microscopy. For the purposes of this invention it is further defined to include bacteria and microscopic fungi. Microscopic fungi is defined to include yeasts.

According to the present invention, there is provided use of a cosmetically acceptable diluent or carrier and an antimicrobially effective amount of high surface area zinc oxide powder having a surface area between 30m²/g and 100m²/g and a particle size between 0.1 and 200µm in diameter for the preparation of a topical antimicrobial composition. A further use is claimed in claim 6.

Preferably the surface area of the high surface area zinc oxide is greater than 90m²/g and the particle size is between 0.1 and 20.5µm.

In a preferred topical formulation, the high surface area zinc oxide is present from 1 to 10%, preferably 3 to 8%, most preferably 4 to 6% by weight of the total composition.

Acne is a common affliction of many people in their teenage years and sometimes beyond. As a result of puberty, teenagers often have increased levels of sebum. The initial inflammation of the follicle wall in the development of acne results from the presence of free fatty acids derived from the sebum. The normal bacterial flora in the sebaceous duct produce the enzymes responsible for splitting triglycerides in the sebum and releasing these fatty acids. The main micro-organisms in the sebaceous duct are *Propionibacterium acnes* and one or two species of *Staphylococcus aureus.* Therefore in the presence of excess sebum these micro-organisms result in the development of acne.

Most approaches to a cure for acne focus on trying to absorb the excess sebum or to act on the bacteria present.

High surface area zinc oxide has been found to inhibit the growth of *Staphylococcus aureus* and *Propionibacterium acnes.* By inhibiting their growth, less of the sebum is split into the free fatty acids which act to inflame the follicle wall. It may be used as a bacteriostatic agent. So high surface area zinc oxide may be used to treat acne. High surface area zinc oxide may be used in the preparation of a medicament for the treatment of acne.

Athletes foot is the loose term applied to a skin eruption on the foot, usually between the toes. It is a cutaneous fungal infection, most commonly caused by *Tricophyton rubrum, Tricophyton mentagrophytes* or *Epidermophyton floccosum.* In addition to the effect of the micro-organisms, other factors such as wetness or an increase in temperature can contribute to disease development by providing ideal conditions for the initiation and growth of fungal infections.

The condition is commonly treated by careful foot hygiene, removing the damp conditions helpful to fungal growth and by the use of antifungal agents. High surface area zinc oxide has been found to be a potent antifungal agent. This invention teaches high surface area zinc oxide as a potent antifungal agent and the use of high surface area zinc oxide as a treatment for athletes foot. High surface area zinc oxide may be used in the preparation of a medicament for the treatment of athletes foot.

Dandruff is a common human condition characterised by the excess scaling of scalp tissue. *Pityrosporum ovale* is a yeast whose growth is accelerated as a result of the dandruff condition, resulting in secondary infection and a worsening of the condition.

It has been found that high surface area zinc oxide is a potent agent against microbial growth particularly at inhibiting the growth of *P.ovale* and *S.aureus.* So high surface area zinc oxide may be used to prevent microbial growth occurring during dandruff conditions. High surface area zinc oxide may be used in the preparation of a medicament for the treatment of dandruff.

Nappy rash, the skin eruption which tends to occur on the buttocks of infants is due to infrequent changing of soiled nappies. The condition is often worsened by secondary infection with *Candida albicans.* To prevent this condition, nappies are changed regularly and care is taken to ensure that the baby's bottom is dried properly. The buttocks are commonly treated with an agent to absorb any surplus liquid.

High surface area zinc oxide has been found to be highly efficacious when used in the treatment of nappy rash. It is a potent antifungal, and by stopping the growth of *Candida albicans* it prevents the associated secondary infection.

In an embodiment of the invention, the compositions may be formulated as a gel. Such gels may be formulated in a manner known to those skilled in the art, and may include, but not limited to:-
a) antibacterials such as dichlorobenzyl alcohol, triclosan, chlorhexidine digluconate and salicylic acid;
b) oil absorbers such as silica;
c) alcohols such as denatured ethanol, isopropyl alcohol;
d) humectants such as panthenol, butylene glycol, glycerin and propylene glycol;
e) preservatives such as methyldibromo glutaronitrile, phenoxyethanol, magnesium chloride, magnesium nitrate, methylchloroisothiazolinone, methylisothiazolinone or any paraben, for example such as methylparaben, ethyl paraben and propyl paraben;
f) solubilisers such as polysorbate 20, polyethylene glycol-40 hydrogenated castor oil;
g) gelling agents such as xanthan gum, hydroxyethylcellulose, sodium magnesium silicate;
h) emollients such as glycerin, propylene glycol and butylene glycol.

In a further embodiment of the invention, the antimicrobial formulation may be a skin wash, such as a cleanser, moisturiser, face wash, lotion, stick or cream. The composition may be formulated in a manner known to those skilled in the art. Such compositions may include, though not limited to:-
a) alcohols such as propanol, stearyl alcohol, denatured ethanol.
b) emulsifiers such as steareth-2, glyceryl stearate, hydrogenated vegetable glycerides, steareth-21, ceteth-20, cetyl alcohol, cetearyl alcohol, stearic acid, paraffin, stearyl alcohol, polawax, tribehenin, ceteareth-7, ceteth-5.
c) emollients such as Polypropylene Glycol 5-ceteth-20, methyl gluceth-10, Dicaprytyl maleate, cetearyl isononanoate, silicones, paraffinum liquidum and octyl palmitate petrolatum, dioctyl maleate, isohexadecane, cetearyl octanoate and isopropyl myristate.
d) solubilisers such as polysorbate 80, polysorbate 20, polyethylene glycol-40 hydrogenated castor oil, any polysorbate.
e) antibacterials such as triclosan, chlorhexidine digluconate, salicylic acid and dichlorobenzyl alcohol.
f) thickeners such as hydroxyethylcellulose, xanthan gum, sodium magnesium silicate, magnesium aluminium silicate and cellulose.
g) detergents such as sodium laureth sulfate, ammonium lauryl sulfate, magnesium lauryl sulfate, disodium undecylenamido MEA-sulfosuccinate.
h) preservatives such as phenoxyethanol , 2-bromo-2-nitropropane-1,3-diol, methyldibromo glutaronitrile, imidazolidinyl urea, magnesium chloride, magnesium nitrate, methylchloroisothiazolinone, methylisothiazolinone, or any paraben, for example such as propyl paraben, butyl paraben, ethyl paraben, methyl paraben.
i) absorbents such as hydrated silica, clays, talcs.
j) antioxidants such as butylated hydroxytoluene or butylated hydroxyacetone.
k) moisturisers such as butylene glycol, propylene glycol, sorbitol and glycerin, panthenol, sodium hyaluronate, sodium PCA.

In another embodiment of the invention the composition may be formulated as a shampoo. Such a composition may be formulated in a manner known to those skilled in the art, and may include, but not limited to:-
a) surfactants such as cocamidopropyl betaine and sodium laureth sulphate;
b) thickeners such as xanthan gum, hydroxyethylcellulose, laureth 3, sodium chloride, polyethylene glycol-55 propylene glycol oleate and polyethylene glycol-40 hydrogenated castor oil;
c) pearlising agents such as formaldehyde, stearic acid, cocamide MEA, glycol distearate glycol stearate and methyldibromo glutaronitrile;
d) solubilisers such as laureth 3, polyethylene glycol-40 hydrogenated castor oil, polyethylene glycol-55 propylene glycol oleate and propylene glycol;
e) conditioners such as polyquaternium-39, polyquaternium-7, polyquaternium-10 and hydroxypropyl guar hydroxypropylthrimonium chloride;

The efficacy of high surface area zinc oxide as an antimicrobial agent has been demonstrated by *in vivo* and *in vitro* trials. The suitability of high surface area zinc oxide for the purposes stated herein has been demonstrated by trials of standard control formulations against those formulations containing high surface area zinc oxide.

The invention is further disclosed by way of the following, non limiting examples. Unless otherwise stated, the zinc oxide used in the following formulations has a surface area greater than 90m²/g and the particles have an average diameter of 10.47 µm.

### Example 1 - Skin Treatment Gel + High Surface Area Zinc Oxide

| Ingredients | % |
|---|---|
| Alcohol (denatured) | 10.00 |
| Allantoin | 0.10 |
| Glycerin | 1.00 |
| Butylene Glycol | 4.00 |
| Xanthan Gum | 1.00 |
| Phenoxyethanol | 0.20 |
| Hydrated silica | 0.50 |
| Dichlorobenzyl alcohol | 0.10 |
| Colour | qs |
| Benzophenone - 4 | 0.10 |
| Purified Water | to 100 |
| Panthenol | 0.50 |
| High Surface Area Zinc Oxide | 5 |

Xanthan gum, dispersed in 2% of the butylene glycol was added to some of the purified water, and mixed together for 30 minutes. Allantoin, sequestrene and panthenol were added and the mixture was then stirred for 5 minutes. The mixture was cooled to 35°C, then premixed phenoxyethanol and glycerin was added to the mixture, followed by premixed alcohol (denatured) and purified water, followed by premixed dichlorobenzyl alcohol and butylene glycol. The mixture was then stirred. Benzophenone-4 and water were then added with stirring, followed by hydrated silica and high surface area zinc oxide. The mixture was stirred, cooled to below 35°C and then the colour was added. Cold water was added to the bulk, and the mixture stirred for a further 30 minutes.

### Example 2 - Non-oily Moisturiser + High Surface Area Zinc Oxide

| Ingredients | % |
|---|---|
| High Surface Area Zinc Oxide | 5 |
| Colour | qs |
| Perfume | 0.10 |
| Triclosan | 0.10 |
| Allantoin. | 0.10 |
| Phenoxyethanol | 0.20 |
| Hydroxyethylcellulose | 2.00 |
| Polysorbate 20 | 1.00 |
| Butylene Glycol | 3.50 |
| Glycerin | 4.50 |
| Purified Water | to 100 |

### Stage 1

Hydroxyethylcellulose was added to purified water and then homogenised for at least 30 minutes. The homogeniser was switched off and with stirring allantoin and phenoxyethanol, which had been previously dissolved in glycerin and butylene glycol, were added.

### Stage 2

Butylene glycol and glycerin were warmed together to 45°C. Then with stirring, triclosan and high surface area zinc oxide were added, stirred and completely dissolved and cooled to 35°C.

### Stage 3

Using a homogeniser, stage 2 was added to stage 1 and homogenised for 10 minutes. The perfume, previously dispersed in polysorbate 20 was then added and stirred in well. The colour was added and the emulsion was then homogenised for a further 5 to 10 minutes until the product was smooth. Purified water, sufficient to make the formulation up to bulk, was then added.

### Example 3 - Cleansing Lotion + High Surface Area Zinc Oxide

| Ingredients | % |
|---|---|
| High Surface Area Zinc Oxide | 5.00 |
| Purified Water | to 100 |
| Colours | qs |
| Melaleuca alternifolia | 0.50 |
| Polypropylene- 5-ceteth- 20 | 3.25 |
| Polysorbate 80 | 0.20 |
| Citric acid | 0.12 |
| Disodium Phosphate | 0.38 |
| Triclosan | 0.30 |
| Butylene Glycol | 0.20 |
| Alcohol (denatured) | 48.00 |

### Stage 1

Alcohol (denatured) and triclosan were mixed together until homogeneous. Water was added and mixed well. Butylene glycol was then added and the mixture stirred.

### Stage 2

To a suitable stainless steel container disodium phosphate and water were added and warmed to 55-60°C with stirring. Then some water was added, with stirring and the mixture was then allowed to cool.

### Stage 3

When the temperature of stage 2 had reached 20-25°C it was added to stage 1 with stirring. Citric acid was added and mixed well. To a suitable stainless steel container polypropylene glycol-5-ceteth-20, melaleuca alternifolia and high surface area zinc oxide were added. This was then mixed thoroughly and added to the main vessel. In a suitable container polypropylene glycol-5-ceteth-20 and polysorbate 80 were premixed and this was then added to the main vessel. The colour was added, followed by water to make up to bulk.

### Example 4 - Cleansing Wash + High Surface Area Zinc Oxide

| Ingredients | % |
|---|---|
| Cocamidopropyl betaine 30% | 5.00 |
| Benzophenone - 4 | 0.10 |
| Sodium citrate | 0.60 |
| Disodium undecylenamido MEA-sulfosuccinate solution | 1.00 |
| Triclosan | 0.20 |
| Salt | 1.00 |
| Laureth - 3 | 2.00 |
| Sodium laureth sulphate | 47.20 |
| Melaleuca alternifolia | 0.50 |
| Phenoxyethanol | 0.15 |
| Colour | qs |
| Citric acid | 0.10 |
| Purified Water | to 100 |
| High Surface Area Zinc Oxide | 5.00 |

### Stage 1

In a vessel sodium laureth sulphate, metaleuca alternifolia and high surface area zinc oxide were mixed until uniform.

### Stage 2

In the base pan the triclosan was dispersed in cocamidopropyl betaine 30% and stirred for 5 minutes. The purified water was added and stirred well. Citric acid was added and stirred until dissolved followed by sodium citrate, which was stirred until dissolved and then stirred for a further 10 minutes. The mixture was cooled to 30-35°C. Then premixed phenoxyethanol in water, followed by benzophenone-4 in water were added. The colour was added followed by saline solution and water to make up to bulk.

### Example 5 - Moisture Fluid + High Surface Area Zinc Oxide

| Ingredients | % |
|---|---|
| High Surface Area Zinc Oxide | 5.00 |
| Glyceryl stearate | 1.50 |
| Steareth-2 | 2.00 |
| Steareth-21 | 1.00 |
| Cetyl Alcohol | 1.00 |
| Glycerin | 1.00 |
| Butylene Glycol | 2.00 |
| Purified Water | 86.50 |

### Stage 1

Glyceryl stearate, steareth-2, steareth-21, cetyl alcohol and high surface area zinc oxide were melted together at 70-75°C.

### Stage 2

Glycerin was dissolved with stirring, in water at 70-75°C.

### Stage 3

Stage 1 was then added to stage 2 with stirring and then homogenised for 15 minutes. Water was added to the stirred mixture, which was subsequently cooled to 35°C. Butylene glycol was added and the mixture stirred until homogeneous and then made up to bulk with water.

### Example 6 - Anti Dandruff Shampoo + High Surface Area Zinc Oxide

| Ingredients | % |
|---|---|
| Magnesium aluminium silicate | 0.30 |
| Polyacrylic acid solution | 1.50 |
| Purified water | 60.10 |
| High Surface Area Zinc Oxide | 2.00 |
| Preservative | 0.08 |
| Salt | 1.00 |
| Citric acid | 0.02 |
| Cocamidopropyl betaine 50% | 5.00 |
| Sodium laureth sulphate | 30.00 |

Citric acid, salt and preservative were dissolved in water. Polyacrylic acid solution and magnesium aluminium silicate were added and the mixture homogenised. The mixture was then stirred for 20 minutes. High surface area zinc oxide, sodium laureth sulphate and cocamidopropyl betaine were added, followed by sufficient salt to obtain the correct viscosity.

### Example 7 - Mens Facial Wash + High Surface Area Zinc Oxide

| Ingredients | % |
|---|---|
| High Surface Area Zinc Oxide | 5.00 |
| Hydroxyethylcellulose | 1.25 |
| Sodium laureth sulfate | 6.57 |
| Disodium undecylenamido MEA-sulfosuccinate | 1.00 |
| Butylene Glycol | 2.00 |
| Preservative | 0.80 |
| Benzoic acid | 0.10 |
| Polysorbate 20 | 2.00 |
| Perfume | 0.40 |
| Herbal extract | 0.60 |
| Colour | qs |
| Purified Water | to 100 |

### Stage 1

Butylene glycol and preservative were put in a stainless steel vessel and then mixed until uniform.

### Stage 2

Perfume, polysorbate 20 and high surface area zinc oxide were put in another container and mixed until uniform.

### Stage 3

Hydroxyethylcellulose was mixed with some of the water in a stainless steel vessel for 20-30 minutes until fully dispersed. Stage 1 was added, mixed and then the herbal extract, benzoic acid and colour were added and then mixed until fully dispersed.

### Stage 4

Sodium laureth sulfate and disodium undecylenamido MEA-sulfosuccinate were added to stage 3 and mixed. Stage 2 was then added and mixed throroughly. Cold water was added to make up to bulk. The bulk was stirred carefully to prevent foaming.

### Example 8 - Translucent Complexion Base + High Surface Area Zinc Oxide

| Ingredients | % |
|---|---|
| High Surface Area Zinc Oxide | 5.00 |
| Purified Water | 46.68 |
| Triethanolamine pure solution 80% | 1.50 |
| Methylparaben | 0.20 |
| Polypropylene glycol-5-ceteth -20 | 0.60 |
| Allantoin | 0.10 |
| Hydrated silica | 12.00 |
| Stearic acid | 12.00 |
| Cetyl Alcohol | 1.20 |
| Butylene Glycol | 10.00 |
| Propylparaben | 0.10 |
| Dicaprylyl maleate | 4.00 |
| Alcohol denatured | 2.00 |
| Sodium C8-16 isoalkylsuccinyl lactoglobulin sulfonate | 1.00 |
| Alpha-glucan oligosaccharide | 0.20 |
| Herbal extract | 0.40 |
| Octyl palmitate | 3.00 |
| Butylated hydroxytoluene | 0.02 |

### Stage 1

Butylene glycol, allantoin, polypropylene glycol-5-ceteth 20 and methylparaben were added, whilst stirring, to the water at 70-75°C. Hydrated silica and high surface area zinc oxide was. then gradually added and mixed until uniform.

### Stage 2

Stearic acid, cetyl alcohol, octyl palmitate, dicaprylyl maleate were melted together at 70-75°C. Using a silverson, butylated hydroxytoluene, propylparaben and triethanolamine pure solution 80% were added and the mixture stirred for 5 minutes.

### Stage 3

Both stages were warmed to 70-75°C, then stage 2 was added to stage 1, and stirred for 5-10 minutes. The mixture was cooled to 40°C with stirring, then dicaprylyl maleate was added. The mixture was cooled to 30°C then alpha-glucan oligosaccharide in water, denatured alcohol, sodium C8-16 isoalkylsuccinyl lactoglobulin sulfonate, herbal extracts and water were added to the mixture. The mixture was then stirred until uniform.

### Example 9 - Pressed Powder + High Surface Area Zinc Oxide

| Ingredients | % |
|---|---|
| High Surface Area Zinc Oxide | 5.00 |
| Sanitised talc | 85.72 |
| Magnesium Stearate | 5.00 |
| Methylparaben | 0.10 |
| Red colour | 0.23 |
| Yellow colour | 0.45 |
| Paraffinum liquidum | 2.10 |
| Petrolatum | 1.40 |

Sanitised talc, magnesium stearate, methylparaben, high surface area zinc oxide and colours were mixed together for 10 minutes at high speed. Paraffinum liquidum and petrolatum were mixed together, heated to 75°C then sprayed into the bulk mixture at low speed. The bulk was mixed for 5 minutes, then passed twice through a hammer mill before being passed through a 30 mesh seive.

### Example 10 - Cover Up Stick + High Surface Area Zinc Oxide

| Ingredient | % |
|---|---|
| Chalk | 23.31 |
| Carnauba | 1.29 |
| Candelilla Cera | 1.01 |
| Hydrocarbon was consisting of Cera Microcristallina, paraffin and polyethylene | 4.60 |
| Cera Microcristallina | 4.22 |
| Butylated hydroxyacetone | 0.03 |
| Propylparaben | 0.10 |
| Octyldodecanol | 46.02 |
| Triclosan | 0.19 |
| Allantoin | 0.14 |
| Pigment | 11.98 |
| High Surface Area Zinc Oxide | 5.00 |
| Synthetic wax | 2.01 |

Pigments, high surface area zinc oxide and chalk were added to the Diosna mixer and mixed for 30 minutes. The mix was then passed through the Mikro mill, then a vibrating sieve to give the colour preparation

White wax, Carnauba and candelilla cera were added to a stainless steel steam jacketed pan fitted with a premier dispersator head, and melted together at 90-95°C. To the melt was added the hydrocarbon wax. When melted, ocyldodecanol was added and the mixture stirred.

The mixture was cooled to 85-90°C then propylparaben, butylated hydroxyacetone and triclosan were added to the stirred mixture, followed by allantoin and then by the colour preparation. The mixture was then stirred for a further ten minutes.

The mixture was then stirred through a 40 mesh sieve into a shallow tray and stirred slowly until set.

### Example 11 - Baby Lotion Wipes +High Surface Area Zinc Oxide

| Ingredient | % |
|---|---|
| Sodium citrate | 0.10 |
| Purified water | 70.03 |
| Perfume | 0.10 |
| Polyaminopropyl biguanide | 0.75 |
| 2-Bromo-2-Nitropropane-1,3-Diol | 0.02 |
| Cetrimonium bromide | 0.50 |
| A wax blend consisting of cetearyl alcohol, cetyl palmitate, cocoglycerides and glyceryl stearate | 0.50 |
| Steareth-10 | 1.50 |
| An emulsifier blend consisting of glyceryl stearate and polyethylene glycol-30 stearate | 1.50 |
| Mineral oil | 20.00 |
| High Surface Area Zinc Oxide | 5.00 |

### Stage 1

Steareth 10 and high surface area zinc oxide was added to a base pan containing mineral oil at 70°C. The emulsifier blend and the wax blend were then added to the stirred mixture and melted together at 70°C.

### Stage 2

Cetrimonium bromide was added to purified water at 70°C and mixed in a homogeniser.

### Stage 3

Stage 1 was then added to stage 2, homogenised and stirred. Cold purified water was added and the mixture force cooled to 35°C. The perfume was then added, followed by polyaminopropyl biguanide and a solution of 2-bromo-2-nitropropane-1,3-diol in cold water Water was then added to make up to bulk. Paper wipes were subsequently impregnated with the lotion produced, to give the baby lotion wipes.

### Example 12 - Nappy Rash Cream + High Surface Area Zinc Oxide

| Ingredient | % |
|---|---|
| Cetearyl alcohol | 2.00 |
| High Surface Area Zinc Oxide | 5.00 |
| Arachis Hypogaena | 30.50 |
| Cera Alba | 10.00 |
| Ricinus Communis | 52.50 |

Arachis hypogaena, cera alba and cetearyl alcohol were mixed together in a base pan at 65-70°C. The mixture was then pumped through an 80 mesh sieve into a mixing vessel. High surface area zinc oxide was added to the mixture which was stirred for 5 minutes until homogeneous. Ricinus communis was then added to the mixture and the mixture stirred for 5 minutes until homogeneous.

### Example 13 - Nappy Cream + High Surface Area Zinc Oxide

| Ingredient | % |
|---|---|
| Purified water | 39.40 |
| p-chloro-m-cresol | 0.10 |
| Cetrimonium bromide | 0.50 |
| Cetearyl alcohol | 5.00 |
| Paraffinum liquidum | 40.00 |
| Dimethicone | 10.00 |
| High Surface Area Zinc Oxide | 5.00 |

Cetrimonium bromide and p-chloro-m-cresol were added to a base pan containing water at 60-65°C. A vacuum was applied to a fryma and the contents of the base pan transferred to the mixer via a sieve.

Dimethicone, paraffinum liquidum and cetearyl alcohol were added to the base pan, and warmed to 60-65°C. The contents of the base pan were then transferred to the mixer via a sieve. The contents of the mixer were stirred for 10 minutes. Maintaining the vacuum on the mixer, the stirred contents were cooled to 50-60°C when high surface area zinc oxide was added. The mixture was then stirred until a smooth white cream was produced. The cream was then cooled to 35°C and discharged to storage.

### Example 14- Shower Gel + High Surface Area Zinc Oxide

| Ingredients | % |
|---|---|
| Purified Water | 47.48 |
| Perfume | 0.10 |
| Colour | 0.00075 |
| Salt | 1.19 |
| Dichlorobenzyl Alcohol | 0.50 |
| Butylated Hydroxytoluene | 0.0048 |
| Triclosan | 0.2916 |
| PEG - 7 Glyceryl Cocoate | 2.916 |
| Citric Acid | 0.0216 |
| A preservative blend consisting of: Phenoxyethanol, Butylparaben, Ethylparaben, Methylparaben and Propylparaben | 0.80 |
| Cocamidopropyl Betaine | 5.83 |
| Sodium Laureth Sulfate | 45.89 |
| High Surface Area Zinc Oxide | 5.00 |

High surface area zinc oxide was added to purified water and mixed well. Salt and citric acid were then added and the mixture stirred until both had dissolved. Sodium laureth sulfate, cocamidopropyl betaine, phenoxyethanol, butylparaben, ethylparaben, methylparaben and propylparaben were then added and the mixture stirred.
PEG 7 Glyceryl cocoate, butylated hydroxytoluene, triclosan and dichlorobenzyl alcohol were mixed together and warmed to 45°C. Perfume was then added and the mixture stirred until homogenous.
The two mixtures were then combined and stirred until homogenous. Colour solution was added to the stirred mixture followed by water to make up to bulk. The mixture was then stirred until uniform.

### Example 15 - Acne Lotion + High Surface Area Zinc Oxide

| Ingredients | % |
|---|---|
| Purified Water | 85.115 |
| Benzoyl Peroxide | 6.667 |
| Hydroxyethylcellulose | 1.00 |
| Citric Acid | 1.53 |
| Sodium Hydroxide | 0.6915 |
| High Surface Area Zinc Oxide | 5.00 |

Sodium hydroxide was added to a stirred aqueous solution of citric acid. Hydroxyethylcellulose was then added to the mixture which was then stirred for 30 minutes. Benzoyl peroxide was then added followed by some water. The mix was stirred for 2 minutes then homogenised for 20 minutes under vacuum. High surface area zinc oxide was then added to the mixture and stirred thoroughly. Aqueous sodium hydroxide was then added to the stirred mixture, which was then stirred for a further hour.

### Example 16 - Sensitive Cleansing Pads + High Surface Area Zinc Oxide

| Ingredients | % |
|---|---|
| Purified Water | 68.53 |
| A preservative blend consisting of: Phenoxyethanol Butylparaben, Ethylparaben, Methylparaben and Propylparaben | 0.80 |
| Perfume | 0.05 |
| Polysorbate 20 | 1.02 |
| Sodium Citrate | 0.20 |
| Citric Acid | 0.06 |
| Cetrimoniumbromide | 0.51 |
| Alcohol (denatured) | 17.38 |
| PPG - 5 - Ceteth - 20 | 0.31 |
| Butylene Glycol | 4.60 |
| Glycerin | 1.02 |
| Chlorhexidine Digluconate | 0.51 |
| High Surface Area Zinc Oxide | 5.00 |

Chlorhexidine gluconate, butylene glycol, PPG-5-Ceteth-20, glycerin, cetriumoniumbromide, citric acid, sodium citrate were dissolved in purified water. Denatured alcohol, high surface area zinc oxide and the preservative blend were then added and the mixture stirred. A mixture of Polysorbate 20 and perfume was then added and the mixture stirred. Purified water was added to make up to bulk. The mixture was stirred for 30 minutes then pumped through an 80 mesh sieve to a suitable storage vessel. Rayon/polyester pads were subsequently impregnated with the mixture, to give the sensitive cleaning pads.

### Example 17 - Overnight Gel + High Surface Area Zinc Oxide

| Ingredients | % |
|---|---|
| Purified Water | 50.6 |
| Perfume | 0.05 |
| Triclosan | 0.10 |
| Dichlorobenzyl Alcohol | 0.50 |
| Alcohol (denatured) | 39.50 |
| Hydroxyethylcellulose | 1.25 |
| Glycerin | 3.00 |
| High Surface Area Zinc Oxide | 5.00 |

Hydroxyethylcellulose and glycerin were dispersed in water then transferred to a fryma via a sieve covered with muslim.
Denatured alcohol, dichlorobenzyl alcohol and triclosan were mixed together until homogenous. Perfume and then high surface area zinc oxide were then dispersed in the mixture. The mixture was then transferred to the fryma under vacuum via a sieve. The mixture was stirred for 30 minutes until homogeneous. Water was then added to make up to bulk.

### Example 18 - Emergency Gel + High Surface Area Zinc Oxide

| Ingredients | % |
|---|---|
| Purified Water | 37.00 |
| Hydroxypropyl Methylcellulose | 2.50 |
| Sodium Citrate | 0.30 |
| Alcohol (denatured) | 20.00 |
| Butylene Glycol | 15.00 |
| Propylene Glycol | 18.00 |
| Triclosan | 0.20 |
| Salicylic Acid | 2.00 |
| High Surface Area Zinc Oxide | 5.00 |

Propylene glycol, butylene glycol and ethanol were mixed together. Salicylic acid and triclosan were then dissolved in the mixture. High surface area zinc oxide was then added to the stirred mixture. Hydroxypropyl methylcellulose was then dispersed in the mixture and the mixture was stirred for 30 minutes. Aqueous sodium citrate was then added to the mixture under vacuum and the mixture stirred for a further 30 minutes.
The antimicrobial action of high surface area zinc oxide is illustrated by the following methods given below. The material was tested against three bacteria, implicated in dandruff and acne; *P. ovale, S. aureus* and *P. acnes.* In the following experiments the zinc oxide used has a surface area greater than 90m²/g and an average diameter of 10.47 µm.

### ANTIMICROBIAL ACTION AGAINST P. OVALE, S. AUREUS AND P. ACNES

### Method

The high surface area zinc oxide was dispersed at a maximum concentration of 25% in acetone. Two fold dilutions were prepared in acetone and aliquots of each dilution added to the appropriate agars to give a range of concentrations from 2.5% to 0.16%. Test plates and "no product" controls containing water only were streaked with the test organisms. These were then incubated as appropriate for three days.

The *P.ovale* was incubated on *P.ovale* growth medium, made using:-

| Material | Quantity |
|---|---|
| Malt extract agar | 60g |
| Ox bile - dessicated | 20g |
| Tween 40 | 10.0ml |
| Glycerol mono-oleate | 2.5ml |
| Water | to 1 litre |

This was then incubated aerobically at 37°C for three days.

*S.aureus* was tested on Tryptone Soya Agar which was incubated aerobically for 37°C for two days.

P.acnes was tested on Brain Infusion Agar which was incubated annaerobically at 37°C for three days.

### Results

| | **Water Control** | **Acetone Control** | **High Surface Area Zinc Oxide Concentrations** | | | | |
|---|---|---|---|---|---|---|---|
| | | | **2.5%** | **1.25%** | **0.6%** | **0.31%** | **0.16%** |
| *P.ovale* | Growth | Growth | No Growth | No Growth | No Growth | No Growth | No Growth |
| *P.acnes* | Growth | Growth | No Growth | No Growth | No Growth | No Growth | No Growth |
| *S.aureus* | Growth | Growth | No Growth | No Growth | No Growth | No Growth | No Growth |

Inhibition of growth was observed at the lowest concentration tested (0.16% in agar) for all three test organisms.

### Experiment 2

Determination of Minimum Inhibitory Concentrations (MIC) for High Surface Area Zinc Oxide.

### Method

The MIC of high surface area zinc oxide was determined against *P. ovale, S. aureus* and *P. acnes* using Zinc Oxide BP as a control. MIC is the minimum concentration at which the active material will inhibit the growth of the microorganisms to be tested. The control of Zinc Oxide BP, and the test, high surface area zinc oxide, were suspended in acetone at 12.5% and aliquots were added to the agar to give a range of concentrations from 6250ppm to 98ppm.

The *P.ovale* was incubated on *P.ovale* growth medium, made using:-

| Material | Quantity |
|---|---|
| Malt extract agar | 60g |
| Ox bile - dessicated | 20g |
| Tween 40 | 10.0ml |
| Glycerol mono-oleate | 2.5ml |
| Water | to 1 litre |

This was then incubated aerobically at 37°C for three days.

*S.aureus* was tested on Tryptone Soya Agar which was incubated aerobically at 37°C for two days.

*P.acnes* was tested on Brain Heart Infusion Agar which was incubated anaerobically at 37°C for three days.

### Results

**Table 2**

| **Microorganism** | **High Surface Area Zinc Oxide MIC** | **Zinc Oxide BP Control MIC** |
|---|---|---|
| *P. ovale* | 1562 ppm | 3125 ppm |
| *S. aureus* | 390 ppm | >6250 ppm |
| *P. acnes* | 190 ppm | 195 ppm |

From this we can see that high surface area zinc oxide inhibits the growth of *P.ovale* and *S.aureus* at a lower concentration than the Zinc Oxide BP control. The high surface area zinc oxide inhibits the growth of *P.acnes* at the same concentration as the Zinc Oxide Control.

This means that the high surface area zinc oxide inhibits the growth of these three organisms better, or as well as the Zinc Oxide control. These results were obtained using the neat raw materials.

## Claims

1. The use of a cosmetically acceptable diluent or carrier and an antimicrobially effective amount of high surface area zinc oxide powder, having a surface area between 30m²/g and 100m²/g and a particle size between 0.1 and 200 µm in diameter for the preparation of a topical antimicrobial composition.

2. A use as claimed in claim 1 where the surface area of the high surface area zinc oxide is between 90 and 100m²/g and the particle size is between 0.1 and 20.5 µm in diameter.

3. A use as claimed in claim 1 where the high surface area zinc oxide is present from 1 to 10% by weight of the total composition.

4. A use as claimed in claim 1 where the high surface area zinc oxide is present from 3 to 8% by weight of the total composition.

5. A use as claimed in claim 1 where the high surface area zinc oxide is present from 4 to 6% by weight of the total composition.

6. The use of a cosmetically acceptable diluent or carrier and an antimicrobially effective amount of high surface area zinc oxide powder, having a surface area between 30 m²/g and 10 m²/g and a particle size between 0.1 and 200 µm in diameter for the manufacture of a medicament for the treatment of acne, athletes foot, nappy rash and dandruff.

## Patentansprüche

1. Verwendung eines kosmetisch akzeptablen Verdünnungsmittels oder Trägers und einer antimikrobiell wirksamen Menge von Zinkoxidpulver mit großem Oberflächenbereich mit einem Oberflächenbereich zwischen 30 m²/g und 100 m²/g und einer Partikelgröße zwischen 0,1 und 200 µm im Durchmesser zur Herstellung einer topischen antimikrobiellen Zusammensetzung.

2. Verwendung nach Anspruch 1, wobei der Oberflächenbereich des Zinkoxids mit großem Oberflächenbereich zwischen 90 und 100 m²/g und die Partikelgröße zwischen 0,1 und 20,5 µm im Durchmesser liegt.

3. Verwendung nach Anspruch 1, wobei das Zinkoxid mit großem Oberflächenbereich zwischen 1 und 10 Gew.-% der Gesamtzusammensetzung vorliegt.

4. Verwendung nach Anspruch 1, wobei das Zinkoxid mit großem Oberflächenbereich zwischen 3 und 8 Gew.-% der Gesamtzusammensetzung vorliegt.

5. Verwendung nach Anspruch 1, wobei das Zinkoxid mit großem Oberflächenbereich zwischen 4 und 6 Gew.-% der Gesamtzusammensetzung vorliegt.

6. Verwendung eines kosmetisch akzeptablen Verdünnungsmittels oder Trägers und einer antimikrobiell wirksamen Menge von Zinkoxidpulver mit großem Oberflächenbereich mit einem Oberflächenbereich zwischen 30 m²/g und 100 m²/g und einer Partikelgröße zwischen 0,1 und 200 µm im Durchmesser zur Herstellung eines Medikaments zur Behandlung von Akne, Fußpilz, Windeldermatitis und Schuppen.

## Revendications

1. Utilisation d'un diluant ou d'un agent vecteur acceptable d'un point de vue cosmétique et d'une quantité efficace en matière d'effet antimicrobien d'une poudre d'oxyde de zinc à grande surface spécifique, ayant une surface spécifique située entre 30m²/g et 100m²/g et une taille de particule située entre 0,1 et 200µm de diamètre pour la préparation d'une composition antimicrobienne d'application topique.

2. Utilisation selon la revendication 1, où la surface spécifique de l'oxyde de zinc à grande surface spécifique est située entre 90 et 100m²/g et la taille des particules est située entre 0,1 et 20,5µm en diamètre.

3. Utilisation selon la revendication 1, où l'oxyde de zinc à grande surface spécifique est présent entre 1 et 10% en poids de la composition totale.

4. Utilisation selon la revendication 1, où l'oxyde de zinc à grande surface spécifique est présent entre 3 et 8% en poids de la composition totale.

5. Utilisation selon la revendication 1, où l'oxyde de zinc à grande surface spécifique est présent entre 4 et 6% en poids de la composition totale.

6. Utilisation d'un diluant ou d'un agent vecteur acceptable d'un point de vue cosmétique et d'une quantité efficace en matière d'effet antimicrobien d'une poudre d'oxyde de zinc à grande surface spécifique, ayant une surface spécifique située entre 30m²/g et 100m²/g et une taille de particule située entre 0,1 et 200µm de diamètre pour la fabrication d'un médicament destiné au traitement de l'acné, des mycoses, de l'érythème fessier et des pellicules.
